# EUROPEAN PATENT APPLICATION

(11) **EP 4 289 923 A1**
(43) Date of publication of application: **13.12.2023**
(21) Application number: 22756185.9
(22) Date of filing: 16.02.2022
(51) Int. Cl.: C12M 1/00, C12N 5/00, B65D 81/22

(54) **TRANSPORT CONTAINER FOR PROTECTING TRANSPLANT, AND METHOD OF USING TRANSPORT CONTAINER**

(30) Priority: 16.02.2021 JP 2021022420
(71) Applicant: TERUMO Kabushiki Kaisha, Tokyo 151-0072 (JP)
(72) Inventor: MATSUDA, Isamu, Ashigarakami-gun, Kanagawa 259-0151 (JP); OHASHI, Fumiya, Ashigarakami-gun, Kanagawa 259-0151 (JP)
(74) Representative: Prüfer & Partner mbB Patentanwälte · Rechtsanwälte
(86) International application number: PCT/JP2022/006034
(87) International publication number: WO 2022/176867

(57) **Abstract**

An object of the present invention is to provide a transport container capable of capturing air bubbles remaining in a container with a simple mechanism and a simple operation to protect a graft. The above problem has been solved by a transport container for protecting a graft, the transport container including: a lid body that seals a container for housing a graft, in which the lid body has a protrusion protruding toward a space inside the container in a state of being attached to the container, a substantially annular groove is formed on a lower surface of the protrusion, gas and liquid inside the container housing the graft and liquid are pushed out by the lid body to achieve a liquid-tight state, and air bubbles in the liquid can be captured in the groove on the lower surface.

## Description

### Technical Field

The present invention relates to a transport container for protecting a graft and a method for using the transport container.

### Background Art

In recent years, development of new regenerative medicine has been advanced as a solution to treatment of severe heart failure. As an example thereof, a method for applying a sheet-shaped cell culture produced using a temperature-responsive culture dish to which tissue engineering is applied in severe myocardial infarction or the like to a heart surface has been attempted. The technique using a sheet-shaped cell culture can safely transplant a large amount of cells to a wide range, and is particularly useful for, for example, treatment of myocardial infarction (including chronic heart failure associated with myocardial infarction), dilated cardiomyopathy, ischemic cardiomyopathy, and a heart disease (for example, heart failure, in particular chronic heart failure) associated with systolic dysfunction (for example, left ventricular systolic dysfunction).

For clinical application of such a sheet-shaped cell culture, for example, it is necessary to house a produced sheet-shaped cell culture in a container together with a storage solution and to transfer the sheet-shaped cell culture to an intensive care unit or the like where transplantation is performed. However, the sheet-shaped cell culture has low absolute physical strength, and wrinkles, tears, breakage, and the like occur in the sheet-shaped cell culture due to vibration generated, for example, when the container is transferred. Therefore, a high level of technology is required for the transfer operation, and it is necessary to pay careful attention to the transfer operation.

In order to meet such needs, various methods and containers have been developed. For example, in a membrane-shaped tissue storage/transport container described in Patent Literature 1 below, a storage is filled with a storage liquid to such an extent that no gas layer is formed. Therefore, the storage liquid does not undulate or flow, and as a result, vibration is not transmitted to the membrane-shaped tissue, and breakage of the membrane-shaped tissue can be prevented. Furthermore, with a clip-shaped coupling mechanism that elastically compresses a container main body and a lid member, contrivance is made such that coupling can be performed more easily.

A packaging container that carries a biological sample and the like described in Patent Literature 2 has a lid having a recess protruding in a bottom surface direction of a packaging container main body. The recess has a rim protruding in the bottom surface direction of the packaging container main body, and the rim has an air release hole. At the time of sealing with the lid, air inside the recess moves to the outside through the air release hole. Therefore, in the sample container, a space in which a medium can be moved by vibration, impact, and inclination that occur on the sample container during transport is reduced.

A device for housing a fragile object described in Patent Literature 3 includes a lid member. The lid member has a protrusion protruding upward in a lower space in a state of being attached to a container. A horizontal cross-sectional area of the protrusion decreases as it goes vertically upward. An openable and closable communication mechanism is disposed at a top of the protrusion. Gas and liquid inside the container housing the fragile object and liquid are pushed out by the lid member, whereby a lower space can be made liquid-tight.

### Citation List

### Patent Literature

Patent Literature 1: JP 2012-130311 A
Patent Literature 2: JP 2013-128457 A
Patent Literature 3: WO 2019/017466 A

### Summary of Invention

### Technical Problem

In the device that pushes out gas as described above, air bubbles may remain inside the lid in a process of sealing operation. It is difficult to visually confirm presence or absence of remaining air bubbles during operation, and it is difficult to remove air bubbles when air bubbles are found after sealing.

The present invention has been made in view of such a point, and an object of the present invention is to provide a transport container capable of capturing air bubbles remaining in a container with a simple mechanism and a simple operation to protect a graft.

### Solution to Problem

That is, the present invention relates to the following.
[1] A transport container for protecting a graft, the transport container including: a lid body that seals a container for housing a graft, in which the lid body has a protrusion protruding toward a space inside the container in a state of being attached to the container, a substantially annular groove is formed on a lower surface of the protrusion, gas and liquid inside the container housing the graft and the liquid are pushed out by the lid body to achieve a liquid-tight state, and air bubbles in the liquid can be captured in the groove on the lower surface.
[2] The transport container according to [1], in which the lower surface of the protrusion is a flat surface parallel to a liquid surface inside the container.
[3] The transport container according to [1] or [2], in which a side surface of the protrusion is configured so as to be in close contact with a side surface inside the container.
[4] The transport container according to any one of [1] to [3], in which the substantially annular groove is formed along a peripheral edge of the lower surface.
[5] The transport container according to any one of [1] to [4], in which the substantially annular groove has an inverted trapezoidal cross section.
[6] The transport container according to any one of [1] to [5], further including one or more cut grooves intersecting with the substantially annular groove.
[7] The transport container according to any one of [1] to [6], in which the graft is a sheet-shaped cell culture.
[8] A method for transferring a graft, the method including: a step of providing the transport container according to any one of [1] to [7]; a step of attaching the lid body to a container housing the graft and liquid; and a step of performing transfer.
[9] A method for culturing a graft, the method including: a step of providing the transport container according to any one of [1] to [7]; a step of seeding cells in a container housing a medium; a step of attaching the lid body to the container; and a step of culturing the graft in the container.

### Advantageous Effects of Invention

The present invention can capture air bubbles remaining in a container with a simple mechanism and a simple operation to protect a graft, and therefore has a large advantage in terms of operability and manufacturing cost. In addition, the present invention can be attached to a general general-purpose Petri dish by one-touch operation and does not need to use a special container, and is therefore highly versatile.

In particular, since capture of air bubbles can be achieved by a simple operation of vibrating the container, a graft can be reliably protected without depending on an operator's technique. In addition, even when air bubbles are found in the container during transfer or the like, the air bubbles can be easily captured without removing a lid body, and therefore occurrence of contamination or the like can be minimized.

### Brief Description of Drawings

Fig. 1A is a conceptual diagram illustrating a transport container including a lid body according to a first embodiment. Fig. 1B is a conceptual diagram illustrating a state where the lid body is attached to a container.
Fig. 2 illustrates Example in which a lid body of the present invention is attached to a general-purpose Petri dish.

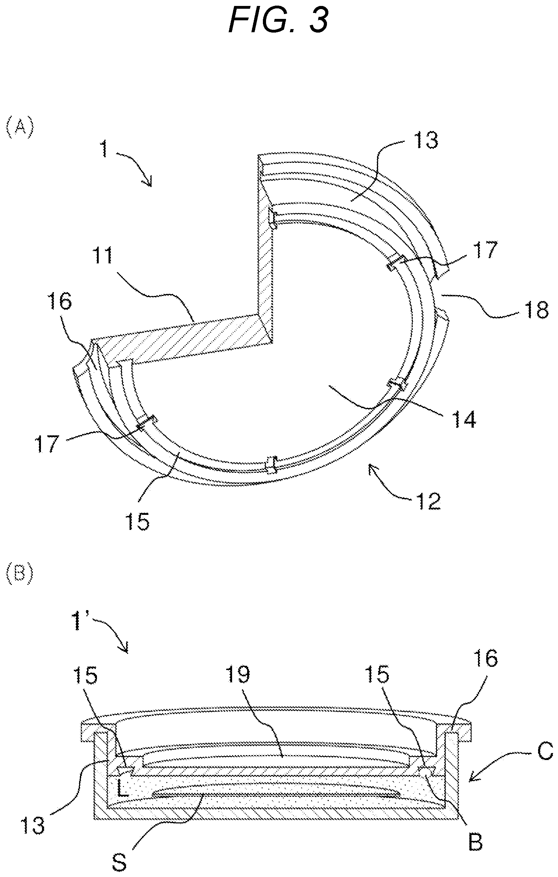

Fig. 3 is a diagram illustrating the transport container of Fig. 1 in detail.

### Description of Embodiments

In the present invention, the "graft" means a structure for transplantation into a living body, and particularly means a structure for transplantation containing living cells as a component. Preferably, the graft is a structure for transplantation that does not contain a structure other than living cells and a substance derived from living cells (for example, a scaffold). Examples of the graft of the present invention include, but are not limited to, a sheet-shaped cell culture, a spheroid, and a cell aggregate. A sheet-shaped cell culture or a spheroid is preferable, and a sheet-shaped cell culture is more preferable.

In the present invention, the "sheet-shaped cell culture" refers to a sheet-shaped culture in which cells are linked to each other. Cells may be linked to each other directly (including via a cellular element such as an adhesion molecule) and/or via an intervening substance. The intervening substance is not particularly limited as long as it is a substance capable of at least physically (mechanically) linking cells to each other, and examples thereof include an extracellular matrix. The intervening substance is preferably derived from a cell, in particular, derived from a cell constituting a cell culture. The cells are linked at least physically (mechanically), but may be further linked functionally, for example chemically or electrically. The sheet-shaped cell culture may be composed of one cell layer (monolayer) or composed of two or more cell layers (laminated (multilayer) body, for example, two layers, three layers, four layers, five layers, or six layers). In addition, the sheet-shaped cell culture may have a three-dimensional structure having a thickness exceeding the thickness of one cell without the cells exhibiting a clear layer structure. For example, in a vertical cross-section of the sheet-shaped cell culture, cells may be present in a state of being non-uniformly arranged (for example, in a mosaic manner) without being uniformly aligned in the horizontal direction.

The sheet-shaped cell culture preferably does not contain a scaffold (support). The scaffold may be used in the technical field in order to attach cells on and/or within a surface thereof and to maintain a physical integrity of the sheet-shaped cell culture. For example, a membrane made of polyvinylidene difluoride (PVDF) is known. However, the sheet-shaped cell culture of the present invention can maintain physical integrity thereof without such a scaffold. In addition, preferably, the sheet-shaped cell culture of the present invention is composed only of substances derived from cells constituting the sheet-shaped cell culture, and does not contain other substances.

The cells constituting the sheet-shaped cell culture are not particularly limited as long as they can form the sheet-shaped cell culture, and include, for example, adherent cells (attaching cells). The adherent cells include, for example, adherent somatic cells (for example, cardiomyocytes, fibroblast cells, epithelial cells, endothelial cells, hepatocytes, pancreatic cells, renal cells, adrenal cells, periodontal ligament cells, gingival cells, periosteal cells, skin cells, synoviocytes, and chondrocytes), and stem cells (for example, myoblasts, tissue stem cells such as cardiac stem cells, embryonic stem cells, pluripotent stem cells such as induced pluripotent stem (iPS) cells, and mesenchymal stem cells). The somatic cells may be stem cells, particularly those differentiated from iPS cells (iPS cell-derived adherent cells). Non-limiting examples of cells that constitute the sheet-shaped cell culture include myoblasts (for example, myoblast cells), mesenchymal stem cells (for example, those derived from bone marrow, adipose tissue, peripheral blood, skin, hair roots, muscle tissue, endometrium, placenta, and cord blood), cardiomyocytes, fibroblast cells, cardiac stem cells, embryonic stem cells, iPS cells, synoviocytes, chondrocytes, epithelial cells (for example, oral mucosal epithelial cells, retinal pigment epithelial cells, and nasal mucosal epithelial cells), endothelial cells (for example, vascular endothelial cells), hepatocytes (for example, hepatocytes), pancreatic cells (for example, islet cells), renal cells, adrenal cells, periodontal ligament cells, gingival cells, periosteal cells, and skin cells. Non-limiting examples of the iPS cell-derived adherent cells include iPS cell-derived cardiomyocytes, fibroblast cells, epithelial cells, endothelial cells, hepatocytes, pancreatic cells, renal cells, adrenal cells, periodontal ligament cells, gingival cells, periosteum cells, skin cells, synovium cells, and chondrocytes.

In the present invention, the container is not particularly limited as long as it can house a graft, liquid, and the like therein and does not leak the liquid, and any container including a commercially available container can be used. Examples of a material of the container include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, and a metal (for example, iron, stainless steel, aluminum, copper, or brass). In addition, the container preferably has at least one flat bottom surface for maintaining the shape of a graft, and examples thereof include, but are not limited to, a Petri dish, a cell culture dish, and a cell culture bottle. The area of the flat bottom surface is not particularly limited, but is typically 1.13 to 78.5 cm², preferably 12.6 to 78.5 cm², and more preferably 9.1 to 60.8 cm².

In the present invention, the liquid inside the container is composed of at least one component, and the component is not particularly limited, but is composed of, for example, a liquid such as water, an aqueous solution, a non-aqueous solution, a suspension, or an emulsion.

The liquid or fluid in the present invention only needs to be a fluid having fluidity as a whole, and may contain a solid substance such as a cell scaffold or another non-liquid component such as air bubbles.

A component constituting the liquid inside the container is not particularly limited as long as it has little influence on a graft. In a case where the graft is a membrane made of a biological material, the component constituting the liquid inside the container is preferably a biocompatible component, that is, a component that does not cause an undesirable action such as an inflammatory reaction, an immune reaction, or an intoxication reaction on biological tissue or cells, or causes such an action at least slightly from a viewpoint of biological stability and long-term storage possibility. Examples of the component include water, physiological saline, a physiological buffer solution (for example, HBSS, PBS, EBSS, Hepes, or sodium bicarbonate), a medium (for example, DMEM, MEM, F12, DMEM/F12, DME, RPMI1640, MCDB, L15, SkBM, RITC80-7, or IMDM), a sugar solution (a sucrose solution, Ficoll-paque (registered trademark) PLUS, or the like), sea water, a serum-containing solution, a lenographin (registered trademark) solution, a metrizamide solution, a meglumine solution, glycerin, ethylene glycol, ammonia, benzene, toluene, acetone, ethyl alcohol, benzol, oil, mineral oil, animal fat, vegetable oil, olive oil, a colloid solution, liquid paraffin, turpentine oil, linseed oil, and castor oil.

In a case where the graft is a sheet-shaped cell culture, preferably, the component constituting the liquid inside the container can stably store cells, contains minimum oxygen, nutrients, and the like necessary for cell survival, and does not destroy the cells by osmotic pressure or the like, and examples thereof include, but are not limited to, physiological saline, a physiological buffer solution (for example, HBSS, PBS, EBSS, Hepes, or sodium bicarbonate), a medium (for example, DMEM, MEM, F12, DMEM/F12, DME, RPMI1640, MCDB, L15, SkBM, RITC80-7, or IMDM), and a sugar solution (a sucrose solution, Ficoll-paque PLUS (registered trademark), or the like).

The amount of the liquid inside the container is not particularly limited as long as the amount of the liquid can hold a graft in a state where the lid body is attached to the container and a liquid volume formed between a bottom surface of the container and a lower surface of the lid body has such a height that does not cause the graft to swing. In an aspect of the present invention, the sheet-shaped cell culture has a diameter of about 35 to 55 mm and an area of 6 cm² or more. The liquid volume is, for example, 1.0 mm to 70.0 mm regardless of the diameter of the sheet-shaped cell culture.

In the present invention, the lid body is not particularly limited as long as it seals the container. Examples of a material of the lid body include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, and a metal (for example, iron, stainless steel, aluminum, copper, or brass).

In the present invention, the shapes of the lid body and the container are not particularly limited as long as the lid body and the container can be engaged with each other, and a sealed space can be formed by such engagement. For example, in a case where the container is a general-purpose Petri dish, the shape of the lid body is preferably circular. In addition, the lid body and/or the container may be made of a light-transmissive material such that a state of a graft housed in the container and presence or absence of air bubbles in the liquid can be confirmed.

In the present invention, the "space inside the container" refers to a space below an opening of the container, that is, a housing space of the container capable of housing liquid, gas, a graft, and the like. The "state of being attached to the container" refers to a state where the lid body is attached to the container to seal the space inside the container.

In the present invention, the "protrusion" refers to a portion protruding downward from a top plate of the lid body. That is, the protrusion refers to a portion that protrudes toward the space inside the container in a state where the lid body is attached to the container and can push out gas and liquid inside the container. The protrusion has a shape that can be fitted into a space above the liquid surface in the space inside the container to achieve a liquid-tight state. For example, in a case where the container to be used is a circular general-purpose Petri dish, the protrusion has a columnar shape, and a side surface of the protrusion is dimensioned so as to be in close contact with a side surface of the general-purpose Petri dish. The protrusion may be hollow or solid. In a case where the protrusion is hollow, the lid body has a recessed cross section, and a lower surface of the protrusion is made light-transmissive. As a result, a state of a graft housed in the container and the position of air bubbles in the liquid can be easily confirmed.

In the present invention, the "lower surface of the protrusion" refers to a lowermost end surface that first comes into contact with a liquid surface when the lid body is attached to the container. That is, the protrusion has a three-dimensional shape having a side surface and a lower surface, and the space inside the container in a state where the lid body is attached to the container is dimensioned so as to be filled with the protrusion and the liquid. In addition, the lower surface of the protrusion is a flat surface parallel to the liquid surface inside the container, and is configured to suppress generation of a water flow when the protrusion comes into contact with the liquid surface. The lower surface of the protrusion is flat except for the groove. Conversely, for example, in a case where the horizontal cross-sectional area of the protrusion is larger (or smaller) as it goes vertically downward, a water flow is generated when the protrusion enters the liquid, and wrinkles, tears, breakage, and the like may occur in the graft in the liquid.

In the present invention, the "groove" refers to a recessed groove formed on the lower surface of the protrusion toward the top plate of the lid body. That is, since air bubbles remaining in the space inside the container float toward the top plate of the lid body, the air bubbles are suitably captured by the groove formed toward the top plate. The "substantially annular groove" includes a continuous groove or a discontinuous groove extending in a circumferential direction of the lower surface. That is, the air bubbles present on the lower surface are surrounded by the substantially annular groove, and can be easily captured by the groove only by vibrating (for example, inclining) the container. In addition, for example, air bubbles that have floated can be naturally captured by the groove only by leaving the container as it is without vibrating the container.

The groove is preferably formed at a peripheral edge of the lower surface so as to be able to capture air bubbles suitably. This makes it possible to surround air bubbles that may remain on the lower surface, thereby facilitating capture. The cross section of the groove includes, but is not limited to, a rectangular shape, a circular shape, a trapezoidal shape, an inverted trapezoidal shape, and the like, but is preferably an inverted trapezoidal shape in order to make it difficult for air bubbles captured once to return to the space inside the container. The groove can have a width of 0.1 mm to 3.0 mm, more preferably 0.8 mm to 1.3 mm.

Hereinafter, a transport container according to a preferred embodiment of the present invention will be described in detail with reference to the drawings. In the present embodiment, description will be given on the assumption that a container is a circular general-purpose Petri dish, and a lid body has a columnar protrusion fitted into a space inside the container of the general-purpose Petri dish.

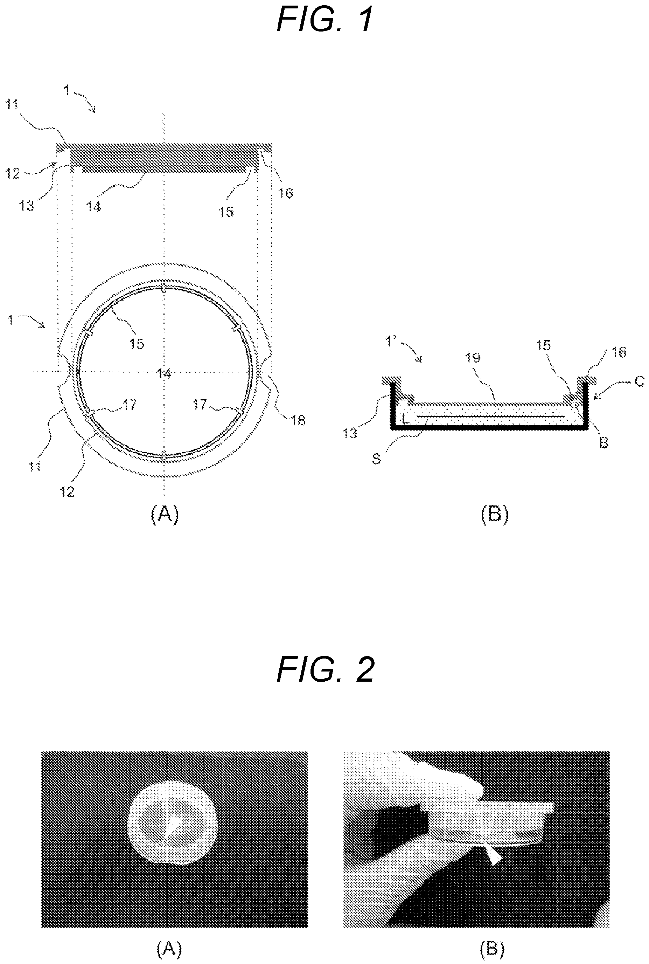

Fig. 1A is a conceptual diagram illustrating a transport container including a lid body according to a first embodiment. Fig. 1B is a conceptual diagram illustrating a state where the lid body is attached to a container. Fig. 2 illustrates Example in which a lid body of the present invention is attached to a general-purpose Petri dish.

As illustrated in Fig. 1, the transport container according to the first embodiment of the present invention includes a lid body 1 that seals a container C for housing a graft S. The lid body 1 includes a top plate 11 and a protrusion 12 protruding downward from the top plate 11. As illustrated in the upper part of Fig. 1A, the protrusion 12 is formed in a solid columnar shape having a side surface 13 and a lower surface 14, and the side surface 13 has a slight draft angle according to an inner surface shape of a general Petri dish. The lower surface 14 of the protrusion 12 is formed as a flat surface parallel to a liquid surface such that generation of a water flow is suppressed when the protrusion 12 comes into contact with the liquid surface.

As illustrated in the lower part of Fig. 1A, the lower surface 14 has a circular shape, and an annular groove 15 is formed along a peripheral edge of the lower surface 14. In addition, the groove 15 has six cut grooves 17 in a direction intersecting with the groove 15. The six cut grooves 17 are arranged so as to be separated from each other at substantially equal intervals in a circumferential direction of the groove 15. Two facing cutouts 18 are formed at an edge of the top plate 11. The top plate 11 has an annular recess 16 so as to surround the protrusion 12 in order to receive an opening edge of the container C (upper part of Fig. 1A).

Fig. 1B illustrates a conceptual diagram in which a lid body 1' as a modification of the lid body 1 of Fig. 1A is attached to the container C. As described above, the protrusion 12 of the lid body 1 is formed in a solid columnar shape, whereas the protrusion 12 of the lid body 1' is formed in a hollow columnar shape. That is, the lid body 1' has a recessed cross section, and the lower surface 14 of the lid body 1' is thinner than that of the lid body 1. Therefore, states of air bubbles and the graft S inside the container C can be visually recognized easily. The top plate 11 is configured to be attached onto an edge of the container C so as to be able to seal the edge of the container C. The height of the top plate 11 decreases in two stages toward a center of the container. A first stage present from the top plate 11 toward the center is an annular flat surface, and a second stage is a circular flat surface. The first stage is present immediately above the groove 15, and the second stage constitutes a gas-permeable membrane 19. The gas-permeable membrane 19 is configured to be able to supply air necessary for culture and quality maintenance of the graft S into the container C in a state where the lid body 1' is attached to the container C.

As illustrated in Fig. 1B, when the lid body 1' is attached to the container C, first, liquid L and the graft S are housed in the container C. At this time, the liquid volume of the liquid L inside the container C is set so as to have such a height that a liquid surface has substantially the same height as the lower surface 14 of the lid body 1' or the liquid surface is slightly higher than the lower surface 14 when the container C is sealed with the lid body 1'. Next, the lid body 1' is attached to the container C, and gas and the liquid L in the space inside the container C are pushed out by the lid body 1' (protrusion 12) to achieve a liquid-tight state. By adjusting the liquid volume as described above, the liquid L is prevented from being excessively pushed out.

Since the protrusion 12 of the lid body 1' is formed so as to be fitted into a space above the liquid surface in the space inside the container C, the side surface 13 of the protrusion 12 is in close contact with an inner surface of the container C. As a result, air bubbles B that may remain are present on the flat lower surface 14, and the air bubbles B are visually confirmed easily. In addition, since the lower surface 14 of the lid body 1' is a flat surface parallel to the liquid surface, it is possible to minimize a water flow generated when the lower surface 14 comes into contact with the liquid surface or moves away from the liquid surface. This is particularly advantageous in a case where the graft S is a very fragile sheet-shaped cell culture.

When the lid body 1' is attached to the container C, an air reservoir is formed (or not formed in some cases) in the annular groove 15 and the cut groove 17. In a case where the air bubbles B remaining on the lower surface 14 of the lid body 1' are found after the attachment operation is completed, the container C (lid body 1') is slightly inclined to move the air bubbles B in a radial direction and attach the air bubbles B to the air reservoir. When the container C is inclined, two fingers (for example, a thumb and a middle finger) are placed on the cutouts 18 from an upper side of the top plate 11, and the container C is lifted so as to be sandwiched between the fingers. As a result, it is possible to prevent an unnecessary force from being applied to the top plate 11 to cause detachment.

The air bubbles B attached to the air reservoir are integrated with (captured by) air in the groove 15, and are prevented from moving to the center where the graft S is present. Even in a case where the air reservoir is not formed, that is, even in a case where the groove 15 is filled with the liquid L, the air bubbles B easily enter the groove 15 and are captured. Since the groove 15 is formed along the peripheral edge of the lower surface 14, that is, so as to surround the air bubbles B on the lower surface 14, the air bubbles B can be integrated with air in the groove 15 regardless of a direction in which the container C (lid body 1') is inclined.

The cut groove 17 serves to block movement of the air bubbles B in a circumferential direction when the container C is inclined to the left and right and the air bubbles B move along the groove 15. In addition, the groove 15 is a linear groove, whereas a cross groove in which the groove 15 and the cut groove 17 intersect with each other is a planar groove, and therefore can more strongly capture the air bubbles B. By forming the cross section of the groove 15 into an inverted trapezoidal shape, it is possible to make it difficult for air (air reservoir, air bubbles B) to move downward. The opening edge of the container C is fitted into the annular recess 16 of the lid body 1' and is therefore hardly detached.

Another aspect of the present invention relates to a method for transferring a graft, the method including: a step of providing the transport container of the present invention; a step of attaching the lid body to a container housing the graft and liquid; and a step of performing transfer.

Transfer of the graft is performed in a state where the graft and the liquid are housed in the container and the lid body is attached. Air bubbles move to the groove in advance by vibration during transfer, or are captured by inclining the container to move the air bubbles to the groove, and are transferred in a state where a graft is protected. The transfer method may include a step of capturing air bubbles in the groove (vibrating the container).

A still another aspect of the present invention relates to a method for culturing a graft, the method including: a step of providing the transport container of the present invention; a step of seeding cells in a container housing a medium; a step of attaching the lid body to the container; and a step of culturing the graft in the container.

The step of seeding cells in a container housing a medium can be implemented, for example, by putting a medium of 10% FBS-DMEM/F12 in UpCell and seeding ells at 2.2 x 10e7 cells/9 mL in a case where the graft is a sheet-shaped cell culture. The step of attaching the lid body to the container can also be performed in a state where culture of the cells has progressed to some extent. The step of culturing the graft in the container includes culturing the graft in a state where the lid body is attached to the container, and can be implemented, for example, by putting the container in an incubator or supplying air through a gas-permeable membrane.

For example, an initial stage of the culture method, that is, the step of providing the transport container, the step of seeding cells in a container housing the medium, the step of attaching the lid body to the container, and the like can be performed in a clean room or the like, and the subsequent steps can be performed outside the clean room. Since air bubbles found outside the clean room can be captured without removing the lid body, and the graft can be cultured outside the clean room, a higher degree of freedom is generated in a transfer step and a manufacturing step.

Furthermore, currently, a sheet-shaped cell culture for which culture has been completed is peeled off from a container and transferred to a transfer container, and the sheet-shaped cell culture is often damaged at this time. However, the present invention makes it possible to perform from culture to transfer in the same container. Therefore, the damage on the sheet-shaped cell culture can be minimized, and the transfer operation can be omitted. In addition, even when air bubbles are found during culture or transfer, the air bubbles can be captured without removing the lid body. The culture method may include a step of capturing air bubbles in the groove.

As described above, the present invention can capture air bubbles remaining in a container with a simple mechanism and a simple operation to protect a graft, and therefore has a large advantage in terms of operability and manufacturing cost. In addition, the present invention can be attached to a general general-purpose Petri dish by one-touch operation and does not need to use a special container, and is therefore highly versatile.

In particular, since capture of air bubbles can be achieved by a simple operation of vibrating the container, a graft can be reliably protected without depending on an operator's technique. In addition, even when air bubbles are found in the container during transfer or the like, the air bubbles can be easily captured without removing a lid body, and therefore occurrence of contamination or the like can be minimized.

Although the present invention has been described with reference to the illustrated embodiment, the present invention is not limited thereto. In the present invention, each configuration can be replaced with any configuration that can exhibit similar functions, or any configuration can be added.

### Examples

A physiological buffer was put in a general-purpose Petri dish (6 cm upcell manufactured by Thermo Fisher Scientific Inc.) so as to have a liquid volume of 5 mm, and a sheet-shaped cell culture was put in the liquid. Then, the transport container (lid body) of the present invention was attached to the general-purpose Petri dish. Remaining air bubbles were conformed, and therefore the container was inclined to guide the air bubbles to the groove. As illustrated in Fig. 2A (photographed from a bottom of the container) and Fig. 2B (photographed from a side surface of the container), air bubbles (distal end indicated by arrow) were captured in the groove, and the air bubbles did not move to a center even when the container was inclined to the left and right.

### Reference Signs List

- C: Container
- L: Liquid
- S: Graft
- B: Air bubbles
- 1, 1': Lid body (transport container)
- 11: Top plate
- 12: Protrusion
- 13: Side surface
- 14: Lower surface
- 15: Groove
- 16: Recess
- 17: Cut groove
- 18: Cutout
- 19: Gas-permeable membrane

## Claims

1. A transport container for protecting a graft, the transport container comprising: a lid body that seals a container for housing a graft, wherein the lid body has a protrusion protruding toward a space inside the container in a state of being attached to the container, a substantially annular groove is formed on a lower surface of the protrusion, gas and liquid inside the container housing the graft and the liquid are pushed out by the lid body to achieve a liquid-tight state, and air bubbles in the liquid can be captured in the groove on the lower surface.

2. The transport container according to claim 1, wherein the lower surface of the protrusion is a flat surface parallel to a liquid surface inside the container.

3. The transport container according to claim 1 or 2, wherein a side surface of the protrusion is configured so as to be in close contact with a side surface inside the container.

4. The transport container according to any one of claims 1 to 3, wherein the substantially annular groove is formed along a peripheral edge of the lower surface.

5. The transport container according to any one of claims 1 to 4, wherein the substantially annular groove has an inverted trapezoidal cross section.

6. The transport container according to any one of claims 1 to 5, further comprising one or more cut grooves intersecting with the substantially annular groove.

7. The transport container according to any one of claims 1 to 6, wherein the graft is a sheet-shaped cell culture.

8. A method for transferring a graft, the method comprising: a step of providing the transport container according to any one of claims 1 to 7; a step of attaching the lid body to a container housing the graft and liquid; and a step of performing transfer.

9. A method for culturing a graft, the method comprising: a step of providing the transport container according to any one of claims 1 to 7; a step of seeding cells in a container housing a medium; a step of attaching the lid body to the container; and a step of culturing the graft in the container.
